# EUROPEAN PATENT APPLICATION

(11) **EP 0 990 902 A1**
(43) Date of publication of application: **05.04.2000**
(21) Application number: 98923129.5
(22) Date of filing: 04.06.1998
(51) Int. Cl.: G01N 33/569, G01N 33/545

(54) **METHOD FOR DETECTING INFECTION WITH ENTEROHEMORRHAGIC $i(ESCHERICHIA COLI)**

(30) Priority: 06.06.1997 JP 14891797
(71) Applicant: Mitsubishi Chemical Corporation, Chiyoda-ku, Tokyo (JP)
(72) Inventor: ONO, Tomoko, Yuka Medias Co., Ltd., Inashiki-gun, Ibaraki 300-0332 (JP); TAKADA, Makoto, Yuka Medias Co., Ltd., Inashiki-gun, Ibaraki 300-0332 (JP); ITO, Akio, Yuka Medias Co., Ltd., Inashiki-gun, Ibaraki 300-0332 (JP)
(74) Representative: VOSSIUS & PARTNER
(86) International application number: JP9802483
(87) International publication number: WO9855871

(57) **Abstract**

A method for detecting infection with enterohemorrhagic *E. coli* which comprises holding enterohemorrhagic *E. coli* lipopolysaccharides on carrier particles, reacting an anti-lipopolysaccharide antibody in samples with the lipopolysaccharides on the carrier particles, and then determining the extent at agglutination of the carrier particles.

## Description

### TECHNICAL FIELD

The present invention relates to a method for detecting infection with enterohemorrhagic *Escherichia coli* and to a reagent kit for use therein.

### BACKGROUND ART

Enterohemorrhagic *Escherichia coli* is characterized by producing Vero toxin which causes hemorrhagic diarrhea and hence is also called Vero toxin producing *Escherichia coli*. Infection with this *Escherichia coli* causes not only hemorrhagic colitis but also acute kidney failure or thrombotic thrombocytepenic purpura (TTP) due to hemolytic-uremic syndrome (HUS) as a sequel for some patients, an eventually resulting in deaths of infants or the aged.

Known serum types of enterohemorrhagic *E. coli* include O157:H7, O157:NM, O26:H11, O91:H21, O111:NM, O113:H21, O145:NM, etc. Among these, O157:H7 is considered important since it is isolated most frequently in our country and its pathogenicity is strong.

The detection ratio of enterohemorrhagic *E. coli* considerably decreases after the administration of antibiotics. Therefore, if the therapy is deferred, patients will present diarrhea or kidney failure and 1 to 10% of them will suffer sequential hemolytic uremic syndrome (HUS), so that high grade remedy such as dialysis or plasma exchange will be necessary and 3 to 4% of them will pass away due to the rhexis of blood vessels.

Therefore, to earlier diagnose the presence or absence of the infection with enterohemorrhagic *E. coli* and prevent the transition of it to a more serious symptom such as hemolytic uremic syndrome (HUS) by an appropriate therapy, it is necessary to first measure the presence or absence of the infection correctly in a short time.

To identify enterohemorrhagic *E. coli*, it is indispensable to examine the Vero toxin productivity of isolated strains. Since the *E. coli* is detected only in the feces from patients so that the incubation of the feces is the most important test. However, the detection ratio decreases as sickness day passes or by administration of antibiotics so that it is important to collect feces in good timing in an early sickness stage and before the administration of antibiotics. Once patients have suffered from the hemolytic uremic syndrome (HUS) or the like serious disease, enterohemorrhagic *E. coli* can be isolated only in about 10% by the incubation of feces. Further, in patients who have suffered from hemolytic uremic syndrome (HUS), an antibody to Vero toxin cannot be frequently detected at a high titer.

On the other hand, an antibody to enterohemorrhagic *E. coli* lipopolysaccharide (LPS) (anti-LPS antibody) comes to be detected in 7 to 10 sickness days and its antibody titer lasts for about 1 month. The increase in this anti-LPS antibody titer is specific to enterohemorrhagic *E. coli* O157.

Bitzan M. *et al.* (Bitzan M. and Karch H., *J. Clin*. *Microbiol., 30*, 1174-1178, 1992) reported an erythrocyte agglutination method as a method for measuring the anti-LPS antibody in blood. This is to practice the diagnosis of hemolytic uremic syndrome (HUS) by coating sheep erythrocytes with enterohemorrhagic *E. coli* O157 LPS, adding an anti-LPS antibody thereto and observing the occurrence of agglutination of erythrocytes. However, this method has the problem that it takes 3 hours before a judgment can be made. On the other hand, Greatorx, J. *et al*. (Greatorx, J. and G. M. Thorne, *J*. *Clin. Microbiol*., 32, 1172-1178, 1994) reported an enzyme-linked immunosorbent assay (ELISA). This method has the problem on the reaction and measurement that the operations are cumbersome and it takes several hours before a judgment can be made.

### DISCLOSURE OF THE INVENTION

The present invention has been made with a view to providing a method for detecting infection with enterohemorrhagic *E. coli* which requires a short analysis time, is convenient, correct and highly sensitive.

The inventors of the present invention have made extensive study to solve the above problem and found that use of agglutination reaction of latex particles carrying enterohemorrhagic *E. coli* lipopolysaccharides enables the detection of infection with enterohemorrhagic *E. coli* speedily, correctly and at a high sensitivity, thus completing the present invention.

That is, the present invention provides a method for detecting infection with enterohemorrhagic *Escherichia coli*, comprising the steps of holding enterohemorrhagic *Escherichia coli* lipopolysaccharides on carrier particles, reacting an anti-lipopolysaccharide antibody in samples with the lipopolysaccharides on the carrier particles, and determining the extent at agglutination of the carrier particles.

According to a preferred embodiment of the present invention, the method is provided such that the carrier particles are latex particles.

According to another embodiment of the present invention, there is provided a reagent kit for use in the detection of infection with enterohemorrhagic *E*. *coli* by comprising at least carrier particles holding enterohemorrhagic *E. coli* lipopolysaccharides, characterized by reacting an anti-lipopolysaccharide antibody in samples with the lipopolysaccharides on the carrier particles, and determining the extent at agglutination of the carrier particles.

According to still another embodiment of the present invention, carrier particles holding enterohemorrhagic *E. coli* lipopolysaccharides are provided.

In the present invention, the enterohemorrhagic *E*. *coli* is preferably O157 and the enterohemorrhagic *E*. *coli* lipopolysaccharide is preferably a O157 lipopolysaccharide.

Hereafter, the present invention will be described in detail.

The carrier particles used in the present invention are not particularly limited so far as they can carry lipopolysaccharides. Usually, fine particles composed of an organic polymer substance or an inorganic substance which is insoluble in a sample solution is used. Examples of fine particles composed of such organic polymer substance include latex particles composed of synthetic polymers such as polystyrene, styrene-maleic acid copolymer, styrene-methacrylic acid copolymer, acrylic acid-styrene copolymer, styrene-acrylic acid-acryl acrylate copolymer, vinyl acetate-acrylic acid copolymer, styrene-butadiene copolymer, polyacrylic acid esters, and polymethacrylic acid esters. Examples of the fine particles composed of the inorganic substance include those composed of glass, silica, alumina, silica-alumina, activated carbon, or carbon. Among these, polystyrene latex particles are particularly preferred. The diameter of carrier particles may be 0.1 to 10 µm and usually the range of 0.1 to 1 µm is preferred.

The lipopolysaccharide (LPS) carried on the above carrier particles is one originating in enterohemorrhagic *E. coli* and no particular limitation is posed thereon so far as it can react with an antibody to enterohemorrhagic *E. coli* lipopolysaccharide which is present in the serum of a patient. The term "enterohemorrhagic *E. coli*" means that it is not limited to one prepared from cells of enterohemorrhagic *E. coli* but that it may be one produced by a recombinant microorganism having introduced therein a gene coding for the lipopolysaccharide (LPS). Specifically, the above lipopolysaccharide (LPS) can be prepared from enterohemorrhagic *E. coli* by, for example, the phenol hot water method by Westphal, V. *et al*. (Westphal, V. and K. Jaun, *Methods Carbohydr. Chem. 5*: 83-91, 1965) as follows.

Dry cells or wet cells of enterohemorrhagic *E. coli* are floated in warm water at about 65 to 68°C and to this is added quantitative 90% phenol heated to about 68°C. The mixture is stirred for about 30 minutes. After cooling to about 4°C, the mixture is centrifuged and the water layer is separated. To the phenol layer is added pure water again and the resulting mixture is heated to about 60°C and stirred for about 30 minutes. After cooling it, the mixture is centrifuged and the water layer is separated. These two water layers are combined and dialyzed against tap water overnight. After the dialysis, the dialyzate is centrifuged and the supernatant is collected. After adding to this a small amount (5 to 10 mg) of sodium acetate, ethanol is added to obtain white precipitates. The precipitates are washed with ethanol and then with acetone and the precipitates are dissolved in pure water to a proportion of 1%. Then, the solution is subjected to ultracentrifugation to obtain crude toxin (lipopolysaccharides) as a sediment.

The above lipopolysaccharides can be easily carried on the carrier particles by a method known per se which is usually used in carrying a biological substance on carrier particles, for example, by a physical absorption method utilizing physical absorption occurring when carrier particles and lipopolysaccharides are mixed, a chemical coupling method in which the carboxyl groups or amino groups on the latex particle surfaces are chemically coupled to the lipopolysaccharides with a coupling agent such as carbodiimide, etc. The lipopolysaccharides may be coupled to the latex particles through a spacer molecule. The lipopolysaccharides may be coupled to a protein such as albumin by the chemical coupling method and then the protein can be coupled to the carrier particles.

The amount of lipopolysaccharide to be carried on the carrier particles is not particularly limited but, in the case of latex particles, it is usually 0.02 to 2 mg, preferably 0.075 to 0.4 mg, per ml of latex is suitable.

The carrier particles holding lipopolysaccharides thus obtained and a sample solution such as blood or serum are mixed to allow the antibody to enterohemorrhagic *E. coli* lipopolysaccharides in the sample to react with the lipopolysaccharides on the carrier particles. Then, the extent at agglutination of the carrier particles occurring due to the reaction is determined to detect the presence or concentration of the above antibody in the sample, and the presence or absence of the infection with enterohemorrhagic *E. coli* is judged.

The above reaction is carried out usually on a slide plate or in a plastic cell or a glass cell. In the case where the reaction is carried out on a slide plate, the extent at agglutination of carrier particles may be determined by visual observation. In the case of the reaction in a cell, light in the range of visible light to near-infrared light, for example, light having a wavelength of usually 400 to 2,400 nm, preferably 600 to 1,000 nm, is irradiated to the reaction mixture and a change in absorbance or change in intensity of scattered light is detected to determine the extent at agglutination of carrier particles. In this case, use of a calibration curve preliminarily prepared enables calculation of the titer or amount (concentration) of the antibody in the sample.

Examples of the apparatus for carrying out such a determination of agglutination include LPIA-S500 Latex Agglutination Fully Automated Latex Immunoassay System of Mitsubishi Chemical Co., COBAS FARA Apparatus and COBAS MIRA Apparatus of Rosche Diagnostics Systems, Inc., and HITACHI 7070 Analyzer of HITACHI, Ltd.

The agglutination reaction of the above carrier particles is usually carried out in a solution such as saline, a suitable buffer solution at a pH on the order of 5.0 to 10.0, for example, a phosphate buffer solution, a borate buffer solution, a Tris buffer solution or the like. To inhibit non-specific coupling of the carrier particles with proteins in the sample, an adsorption inhibitor comprising a suitable detergent, for example, Triton X-100, Tween 20, Tween 80, etc. may be added to the reaction mixture.

The reagent kit for use in detecting infection with enterohemorrhagic *E. coli* involving the above reaction and measurement step, etc. can be prepared in the same manner as a method known *per se* usually used for the preparation of a kit utilizing immune agglutination reaction using carrier particles except for the carrier particles holding lipopolysaccharides. Such a kit is provided in the same composition as kits utilizing usual immune agglutination reaction. That is, the reagent kit of the present invention comprises at least carrier particles holding enterohemorrhagic *E. coli* lipopolysaccharides and further a sample diluent solution and a standard substance as optional elements.

### BRIEF DESCRIPTION OF THE DRAWINGS

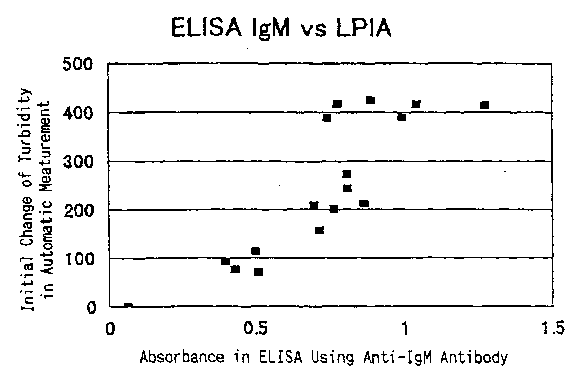

Fig. 1 illustrates a comparison of the results on 17 samples of sera from patients infected with enterohemorrhagic *E. coli*, by enzyme-linked immunosorbent assay (ELISA) conducted in Example 2 and by automatic measurement for anti-LPS antibody using latex particles conducted in Example 3. The horizontal axis indicates absorbance of ELISA and the vertical axis indicates an initial change of turbidity per unit time in automatic measurement using a latex reagent.

### BEST MODE FOR CARRYING OUT THE INVENTION

Hereafter, the present invention will be described in greater detail by examples.

### Example 1 Preparation of latex reagent linked with E. coli O157 LPS

### 〈1〉 Preparation of latex reagent sensitized with E. coli O157 LPS

Study was made using Vero toxin producing *E. coli* O157:H7 strain LPS, which among enterohemorrhagic *E*. *coli* was the most frequently isolated by incubation of feces in Japan. *E. coli* O157 LPS was prepared according to the phenol hot water method of Westphal, V. et al. (Westphal, V. and K. Jaun, *Methods Carbohydr. Chem*. *5*:83-91, 1965) as follows.

40 g of wet cells were floated in 350 ml of warm water at 68°C and quantitative 90% phenol heated to 68°C was added thereto and the mixture was stirred for about 30 minutes. After cooling it to 4°C, the mixture was centrifuged and the water layer was separated. To the phenol layer was added 300 ml of pure water again and the mixture was heated to 60°C and stirred for about 30 minutes. Similarly, after cooling it, the mixture was centrifuged and the water layer was separated. These two water layers were combined and introduced into a dialyzer to dialyze against tap water overnight. After the dialysis, the dialyzate was centrifuged and the supernatant was collected. After adding to this a small amount (5 to 10 mg) of sodium acetate, 10 folds volume of ethanol was added to obtain white precipitates. The precipitates were recovered by centrifugation and subjected to centrifugation washing with ethanol and then with acetone each once to obtain crude toxin (LPS). The crude toxin contained a considerable amount of nucleic acids as contaminants and the precipitates were dissolved in pure water to a proportion of 1% in order to remove the nucleic acids and the solution was subjected to ultracentrifugation (100,000Xg, 1 hour) to obtain sediments. The sediments were dissolved again in pure water and centrifuged. The resulting sediments was dissolved in a small amount of pure water and freeze-thawed to obtain purified endotoxin (yield 2 to 5%). The *E. coli* O157-derived LPS obtained by this method was dialyzed against 0.1 M phosphate buffer solution (pH 7.0) and used in the preparation of the following latex reagent.

### 〈2〉 Preparation of E. coli O157 LPS-sensitized latex

The obtained *E. coli* O157 LPS was immobilized (carried) on the surface of polystyrene latex (Celladyne Corp.) having an average particle diameter of 0.47 µm by the following method to obtain a latex reagent. To 1 ml of a 1% latex solution was added 4 mg of carbodiimide as a coupling agent and reaction was carried out at room temperature for 30 minutes. After centrifugation, the sediments were suspended in 0.1 M phosphate buffer solution (pH 7.0), to which was added 0.1 mg of *E. coli* O157-derived LPS and the reaction was carried out at room temperature for 1 hour. After centrifugation, 30 minute's blocking was carried out with 0.3% bovine albumin. After centrifugation, the sediments were suspended in distilled water to form a latex reagent.

### Example 2 Method for judgment of a slide of agglutination of E. coli O157 LPS-sensitized latex

Saline or normal human serum and rabbit anti-LPS serum were diluted to 20 folds with Tris buffer solution containing BSA to form negative and positive controls, respectively. As test samples, sera of patients were used and diluted in the same manner with Tris buffer solution containing BSA.

100 µl each of the above controls and test samples were added on a slide plate and 35 µl of the latex reagent obtained in Example 1 was added dropwise thereto and mixed on the slide plate. After 3 minutes, the extent of latex particle agglutinates was observed with naked eye.

Test samples were further measured by an enzyme-linked immunosorbent assay (ELISA). The enzyme-Linked immunosorbent assay (ELISA) was practiced as follows. In each well of 96-well Immunoplate (Polysorp, NUNC Co.) was dispensed 100 µl of *E. coli* O157-derived LPS diluted to 1 µg/ml with PBS and incubation was carried out at 37°C for 2 hours. The plate was washed 3 times with 0.05% Tween 20/PBS (PBST) and blocked with 0.01% Tween 20/PBS at 37°C for 1 hour. After washing the plate 3 times, 100 µl each of samples diluted 400 folds with 0.5% BSA/PBST was added and incubated at 37°C for 1 hour. After washing the plate 3 times, 100 µl each of HRP conjugated anti-IgM antibody diluted 2,500 folds with 0.5% BSA/PBST was added and each mixture was incubated at 37°C for 30 minutes. After washing the plate 3 times, 100 µl each of a substrate solution (OPD tablet/citrate buffer solution/aqueous hydrogen peroxide) was added and each mixture was incubated at room temperature for 5 minutes. The reaction was terminated by addition of 100 µl of 2N sulfuric acid solution to the wells, absorbance was measured using an immuno reader (wavelength: 492 nm).

As a result of measurement of 17 test samples from sera of patients who were suspected of infection with *E. coli* O157, 16 test samples were positive in both enzyme-linked immunosorbent assay (ELISA) and latex agglutination method and 1 sample was negative in both, and 100% coincidence was obtained.

### Example 3 Automatic measurement of antibody to E. coli O157 LPS

17 Test samples from the sera of the above patients were used for study. 10 µl of a test sample diluted 40 folds with saline, 180 µl of Tris buffer containing BSA solution, 80 µl of the latex reagent obtained in Example 1 were mixed and automatically added in a reaction cubette. Thereafter, the initial change of turbidity in a near-infrared (800 nm) region was measured for 10 minutes to observe the latex agglutination caused by antigen-antibody reaction. Measurement was made using Latex Agglutination Fully Automated Latex Immunoassay System LPIA-S500.

Fig. 1 illustrates comparison of the results obtained by enzyme-linked immunosorbent assay (ELISA) method and the results of automatic measurement using latex (using LPIA-S500) of 17 test samples of patients' sera. Plotting absorbance of ELISA in horizontal axis and the initial change of turbidity of LPIA-S500 per unit time, positive correlation between the two methods was observed.

### INDUSTRIAL APPLICABILITY

According to the present invention, the presence or absence of the antibody to enterohemorrhagic *E. coli* lipopolysaccharide in serum can be measured in a short time, conveniently, and correctly and based on the results presence or absence of infection with enterohemorrhagic *E. coli* can be detected. The method of the present invention having the above features, unlike the method using patient's feces as a sample in a conventional microbe incubation detection method, uses patient's serum as a sample so that the sample is easy to handle and can be said to be a particularly excellent method for detecting enterohemorrhagic *E. coli*.

## Claims

1. A method for detecting infection with enterohemorrhagic *Escherichia coli*, comprising the steps of:
holding enterohemorrhagic *Escherichia coli* lipopolysaccharides on carrier particles,
reacting an anti-lipopolysaccharide antibody in samples with the lipopolysaccharides on the carrier particles, and
determining the extent at agglutination of the carrier particles.

2. The method as claimed in claim 1, wherein the carrier particles are latex particles.

3. A reagent kit for use in the detection of infection with enterohemorrhagic *Escherichia* comprising at least carrier particles holding enterohemorrhagic *Escherichia coli* lipopolysaccharides, characterized by reacting an anti-lipopolysaccharide antibody in samples with the lipopolysaccharides on the carrier particles, and determining the extent at agglutination of the carrier particles.

4. Carrier particles holding enterohemorrhagic *Escherichia coli* lipopolysaccharides.
